(19) Europäisches Patentamt
European Patent Office
Office européen des brevets

(11) Veröffentlichungsnummer: **0 026 746**
**B1**

(12) # EUROPÄISCHE PATENTSCHRIFT

(45) Veröffentlichungstag der Patentschrift: **10.04.85**

(21) Anmeldenummer: **80810302.2**

(22) Anmeldetag: **26.09.80**

(51) Int. Cl.[4]: **A 61 K 45/06**, A 61 K 37/02, A 61 K 31/70, A 61 K 31/71, A 23 K 1/16, A 23 K 1/17 // (A61K37/02, 31:71),(A61K37/02, 31:43),(A61K37/02, 31:65),(A61K31/71, 31:70)

(54) **Kombinationspräparate zur Anwendung in einem Verfahren zur Steigerung der Wirkung von Antibiotika, antibiotische Präparate mit gesteigerter Wirksamkeit und Verfahren zu deren Herstellung.**

(30) Priorität: **02.10.79 CH 8883/79**

(43) Veröffentlichungstag der Anmeldung: **08.04.81 Patentblatt 81/14**

(45) Bekanntmachung des Hinweises auf die Patenterteilung: **10.04.85 Patentblatt 85/15**

(84) Benannte Vertragsstaaten: **BE CH DE FR GB IT LI LU NL SE**

(56) Entgegenhaltungen:
**DE - A - 2 331 144**
**DE - A - 2 603 122**
**DE - A - 2 655 500**
**FR - A - 2 387 037**
**FR - A - 2 410 476**

**Die Akte enthält technische Angaben, die nach dem Eingang der Anmeldung eingereicht wurden und die *nicht in dieser Patentschrift enthalten sind.***

(73) Patentinhaber: **CIBA-GEIGY AG, Postfach, CH-4002 Basel (CH)**

(72) Erfinder: **Dietrich, Felix M., Prof. Dr., Im Nonnengärtli 18, CH-4102 Binningen (CH)**
Erfinder: **Sackmann, Werner, Dr., Sieglinweg 10, CH-4125 Riehen (CH)**
Erfinder: **Zak, Otokar, Dr., Am Stausee 27/17, CH-4127 Birsfelden (CH)**
Erfinder: **Dukor, Peter, Dr., Augustinergasse 17, CH-4051 Basel (CH)**

**EP 0 026 746 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft Kombinationspräparate zur Anwendung in einem Verfahren zur Steigerung der antibiotischen Wirksamkeit von Antibiotika, welches dadurch gekennzeichnet ist, dass man ein Antibiotikum aus der Gruppe der β-Lactamantibiotika, Aminoglycoside, Tetracycline, Macrolide, Lincomycine, Polyenantibiotika, Polypeptidantibiotika, Anthracycline, Chlor- oder Thiamphenicole, Cycloserine, Fusidinsäuren oder Rifamycine zusammen mit einem Muramylpeptid der Formel

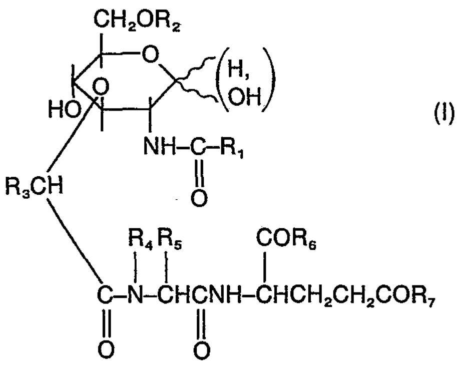

(I)

worin $R_1$ unsubstituiertes oder durch Hydroxy, Niederalkoxy, Niederalkanoyloxy, Halogen, Mercapto, Niederalkylthio, Amino oder Niederalkanoylamino substituiertes Niederalkyl oder Niederalkoxy, oder einen Phenyl- oder Phenylniederalkylrest, wobei der Phenylrest bzw. der Phenylteil gegebenenfalls durch Niederalkyl, Hydroxy, Niederalkoxy oder Halogen und der Niederalkylteil gegebenenfalls durch Hydroxy, Niederalkoxy, Niederalkanoyloxy oder Halogen substituiert ist,

$R_2$ Wasserstoff oder das Acylradikal einer aliphatischen Carbonsäure mit bis zu 30 C-Atomen,

$R_3$ Wasserstoff oder Niederalkyl,

$R_4$ Wasserstoff oder Niederalkyl,

$R_5$ Wasserstoff, unsubstituiertes oder durch Hydroxy, Niederalkoxy, Niederalkanoyloxy, Halogen, Mercapto, Niederalkylthio, Amino, Niederalkanoylamino oder gegebenenfalls durch Niederalkyl, Hydroxy, Niederalkoxy oder Halogen substituiertes Phenyl substituiertes Niederalkyl oder unsubstituiertes oder durch Niederalkyl, Hydroxy, Niederalkoxy oder Halogen substituiertes Phenyl, oder $R_4$ und $R_5$ zusammen Niederalkylen bedeuten, und jeder der Reste $R_6$ und $R_7$ für gegebenenfalls veräthertes Hydroxy oder gegebenenfalls durch Niederalkyl, welches unsubstituiert oder durch Hydroxy, Mercapto, Carboxy, Niederalkoxy, Niederalkylthio, Niederalkoxycarbonyl oder Carbamoyl substituiert ist, substituiertes Amino steht, und/oder einem oder mehreren Salzen von Verbindungen der Formel I mit mindestens einer salzbildenden Gruppe verabreicht, sowie antibiotische Präparate mit verstärkter Wirksamkeit und Verfahren zu deren Herstellung.

Im obengenannten Verfahren verwendet man eine wirksame oder unter-wirksame Dosis des Antibiotikums, je nach Art des letzteren z.B. von etwa 50 bis etwa 500 mg pro Einzeldosis.

Die Muramylpeptide der Formel I werden in Einzeldosen von etwa 5 mg bis ungefähr zur halben Antibiotika-Menge verwendet. Dabei kann das Muramylpeptid-Derivat bis zu 24 Stunden vor oder nach, vorzugsweise jedoch etwa gleichzeitig mit dem Antibiotikum verabreicht werden.

Die Verabreichung der Antibiotika erfolgt auf dem üblichen Wege, wie subkutan, intravenös oder oral, während man die Muramylpeptide, insbesondere wenn sie getrennt von den Antibiotika verabreicht werden, meist subkutan verabfolgt.

Bei diesem Verfahren kann man einzelne Antibiotika wie auch Antibiotikagemische verwenden.

Als bevorzugte Antibiotika seien unter den β-Lactamen die Penicilline, Cephalosporine, Peneme, Nocardicine, Thienamycine und Clavulansäuren genannt.

Penicillin-Antibiotika sind in erster Linie Amoxycillin, Ampicillin, Carbenicillin, Cloxacillin, Cyclacillin, Dicloxacillin, Mecillinam, Methicillin, Penicillin G, Penicillin V, Pivampicillin, Sulbenicillin, Azlocillin, Ticarcillin, Mezlocillin, Pivmecillinam oder 6-(4-endo-Azatricyclo[5.2.2.0$^{2,6}$]undec-8-enyl)-methylenaminopenicillansäure genannt.

Aus der Gruppe der Cephalosporine können z.B. Cefaclor, Cefazaflur, Cefazolin, Cefadroxil, Cefoxitin, Cefuroxim, Cephacetril, Cephalexin, Cephaloglycin, Cephaloridine, Cephalotin, Cefamandol, Cephanon, Cephapirin, Cefatrizin, Cephradin, Cefroxadin (7β-[D-2-amino-2-(1,4-cyclohexadienyl)-acetamido]-3-methoxy-3-cephem-4-carbonsäure = CGP 9000), Cefsulodin, Cefotaxim, Cefotiam, Ceftezol oder Cefazedon genannt werden.

Unter den Nocardicinen ist z.B. Nocardicin A und unter den Thienamycinen und Clavulansäuren z.B. Thienamycin bzw. Clavulansäure zu erwähnen.

Unter den Aminoglycosiden sind insbesondere Streptomycine, z.B. Streptomycin und Streptomycin A, Neomycine, z.B. Neomycin B, Tobramycine, z.B. Tobramycin oder Dibekacin, Kanamycine (z.B. Gemische von Kanamyxin A, B und C), sowie Amicacine, Gentamycine (z.B. Gemische von Gentamycin A, $C_1$, $C_2$ oder $C_{1a}$), oder Sisomicine, wie Sosomicin oder Netilmicin, ferner Lividomycin, Ribocamycin und Paromomycin zu erwähnen.

Als Tetracycline sind insbesondere Tetracyclin, Doxycyclin, Chlortetracyclin, Oxytetracyclin oder Methacyclin zu nennen.

Als Macrolide sind z.B. Maridomycin, Spiramycine, wie Spiramycin I, II und III, Erythomycine, z.B. Erythromycin, Oleandomycine, z.B. Oleandomycin und Tetraacetyloleandomycin, und als Lincomycine z.B. Lincomycin und Clindamycin zu erwähnen.

Als Polyen-Antibiotika sind besonders Amphothericin B und dessen Methylester oder Nystalin zu nennen.

Als Polypeptid-Antibiotika können z.B. Colistin, Gramicidin S, Polymyxin B, Virginamycin, Tyrothricin, Viomycin oder Vancomycin besonders genannt werden.

Als Rifamycine kommen in erster Linie das Rifamycin S, Rifamycin SV oder Rifamycin B oder

deren halbsynthetische Derivate, insbesondere das Rifampicin, in Frage.

In den Muramylpeptiden der Formel 1 haben die allgemeinen Begriffe insbesondere die folgende Bedeutung:

Eine aliphatische Carbonsäure ist z. B. eine gegebenenfalls substituierte Alkan- oder Alkencarbonsäure. In Acylresten von aliphatischen Carbonsäuren sind Substituenten insbesondere gegebenenfalls funktionell abgewandeltes, wie gegebenenfalls veräthertes oder verestertes Hydroxy, z. B. Hydroxy, Niederalkoxy, Niederalkanoyloxy oder Halogen.

Veräthertes Hydroxy $R_6$ oder $R_7$ ist z. B. durch einen aliphatischen Rest veräthertes Hydroxy, wie gegebenenfalls substituiertes Niederalkoxy.

Im vorliegenden Zusammenhang enthalten mit «nieder» bezeichnete Reste und Verbindungen bis und mit 7, in erster Linie bis und mit 4 Kohlenstoffatome. Substituierte Reste können einen oder mehrere, gleiche oder verschiedene Substituenten aufweisen.

Im vorliegenden Zusammenhang können die obengenannten Allgemeindefinitionen folgende Bedeutungen haben:

Niederalkyl ist z. B. Methyl, Äthyl, n-Propyl, Isopropyl, n-Butyl, Isobutyl oder tert.-Butyl, ferner n-Pentyl, Isopentyl, Neopentyl, n-Hexyl oder n-Heptyl.

Phenylniederalkyl ist z. B. Benzyl oder 1- oder 2-Phenyläthyl.

Niederalkoxy ist z. B. Methoxy, Äthoxy, n-Propyloxy, Isopropyloxy oder n-Butyloxy.

Phenylniederalkoxy ist z. B. Benzyloxy oder 1- oder 2-Phenyläthoxy.

Alkanoyloxy ist u. a. Niederalkanoyloxy, z. B. Acetyloxy, Propionyloxy oder Butyryloxy, ferner höheres Alkanoyloxy, z. B. Lauroyloxy, Myristinoyloxy, Palmitoyloxy, Stearoyloxy oder Behenoyloxy.

Halogen steht z. B. für Fluor, Chlor oder Brom.

Niederalkanoylamino ist z. B. Acetylamino oder Propionylamino.

Niederalkylthio ist z. B. Methylthio oder Äthylthio, während höheres Alkylthio z. B. Tetradecylthio, Hexadecylthio oder Octadecylthio bedeutet.

Der Acylrest einer Alkancarbonsäure ist u. a. Niederalkanoyl, z. B. Acetyl, Propionyl, Butyryl oder Hexanoyl, oder höheres Alkanoyl, z. B. Lauroyl, Myristinoyl, Palmitoyl, Stearoyl oder Behenoyl, während z. B. durch Hydroxy substituiertes höheres Alkanoyl u. a. Mycoloyl sein kann.

Der Acylrest einer Alkencarbonsäure ist u. a. Niederalkenoyl, z. B. Allylcarbonyl, oder höheres Alkenoyl, z. B. Undecenoyl.

Niederalkylen ist geradkettig oder verzweigt und ist z. B. Methylen, Äthylen, 1,2-Propylen, 1,3-Propylen oder 1,6-Hexylen.

Durch Niederalkyl substituiertes Amino, das im Niederalkylteil gegebenenfalls substituiert sein kann, ist u. a. Niederalkylamino, z. B. Methylamino oder Äthylamino, oder Carboxyniederalkyl-amino, z. B. Carboxymethylamino.

Niederalkoxycarbonyl ist z. B. Methoxycarbonyl oder Äthoxycarbonyl.

Die Verbindungen der Formel I können in Form von Isomerengemischen oder reinen Isomeren vorliegen. Vorzugsweise liegt der mit dem Sauerstoffatom verknüpfte Rest der Formel $-CH(R_3)-(C=O)-$, falls $R_3$ für Niederalkyl steht, in optisch aktiver Form vor und hat insbesondere die D-Form, während der Rest der Aminosäure der Formel $-B(R_4)-CH(R_5)-C(=O)-$, falls $R_5$ von Wasserstoff verschieden ist, ebenfalls vorzugsweise in optisch aktiver, in erster Linie in der L-Form vorliegt, und der terminale α-Amino-glutarsäurerest vorzugsweise in optisch aktiver, insbesondere in der D-Form, vorliegt. Ferner kann die gegebenenfalls substituierte 1-Hydroxygruppe die α- oder die β-Konfiguration haben; die neuen Verbindungen der Formel I können jedoch auch als Gemisch der 1α und 1β-Isomeren vorliegen.

Salzbildende Gruppen in den Verbindungen der Formel I sind z. B. gegebenenfalls vorhandene Carboxylgruppen. Verbindungen der Formel I mit solchen Gruppen können deshalb in Form von Salzen, insbesondere pharmazeutisch verwendbaren Salzen, wie Metall-, insbesondere Alkali- oder Erdalkalimetall-, z. B. Natrium-, Kalium-, Calcium- oder Magnesiumsalzen, oder von Ammoniumsalzen, z. B. mit Ammoniak oder organischen Aminen, wie Niederalkylaminen, z. B. Triäthylamin, vorliegen. Verbindungen der Formel I mit Aminogruppen, beispielsweise im Rest $R_5$, können als Säureadditionssalze vorliegen. Zur Addition geeignet sind vor allem schwache und pharmazeutisch verwendbare Säuren.

Besonders wertvoll im Rahmen der vorliegenden Erfindung sind Verbindungen der Formel I, in welcher $R_2$ Wasserstoff darstellt und die anderen Reste die obgenannte Bedeutung besitzen, und deren Salze.

Hervorzuheben sind insbesondere Verbindungen der allgemeinen Formel

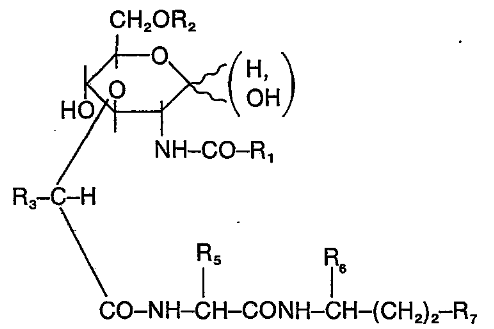

(II)

worin $R_1$ Niederalkyl oder Phenyl, $R_2$ Wasserstoff oder langkettiges Alkanoyl, $R_3$ Wasserstoff oder Methyl, $R_5$ Wasserstoff, Niederalkyl oder Hydroxymethyl, $R_6$ Carbamoyl und $R_7$ Carboxyl bedeuten, und deren Salze.

In erster Linie sind Verbindungen der Formel II zu erwähnen, worin $R_1$ Niederalkyl oder Phenyl, $R_2$ Wasserstoff, $R_3$ Wasserstoff oder Methyl, $R_5$ Wasserstoff, Methyl oder Hydroxymethyl, $R_6$ Carbamoyl und $R_7$ Carboxyl bedeuten, und deren Salze, sowie solche Verbindungen der Formel II, worin $R_1$ Methyl, $R_2$ Wasserstoff oder Stearoyl, $R_3$ Wasserstoff oder Methyl, $R_5$ Methyl, $R_6$ Carbamoyl und $R_7$ Carboxy bedeuten, und deren Salze.

Die zur Herstellung der erfindungsgemässen Kombinationspräparate verwendeten Muramylpeptide sind bekannt oder können nach an sich bekannten Methoden hergestellt werden, siehe z. B. Deutsche Offenlegungsschrift Nr. 2 655 500. In dieser Offenlegungsschrift heisst es auf Seite 28, Mitte, dass die Muramylpeptide «vorzugsweise auch in Kombination mit antiinfektiösen Antibiotika, Chemotherapeutika oder anderen Heilverfahren verabreicht werden» können, «um immunologischen Schädigungen entgegenzuwirken.

Demgegenüber ist die Lehre der vorliegenden Anmeldung, wonach durch Kombination bestimmter Antibiotika mit Muramylpeptiden die Wirkung der Antibiotika verstärkt wird, neu und erfinderisch.

Die DE-A-2 331 144 hat wasserlösliche Extrakte von Mycobakterien mit einem Molekulargewicht zwischen 3500 und 30 000 zum Gegenstand. Hiervon sind die Muramylpeptide der vorliegenden Anmeldung strukturell stark verschieden, wie schon aus ihrem viel niedrigeren Molekulargewicht hervorgeht.

Analoges gilt für die FR-A-2 387 037, die die Verwendung eines stickstoffhaltigen Polysaccharids mit einem Molekulargewicht zwischen 5000 und 300 000 beschreibt.

Die FR-A-2 410 476 beschreibt die Verwendung von durch Extraktion aus Bakterien gewonnenen Peptidoglykanen zur Stimulation der Immunabwehr gegen Tumorerkrankungen. In Anspruch 7 heisst es, dass die pharmazeutischen Präparate neben einem Peptidoglykan auch ein Antiseptikum enthalten können. Als Beispiele für solche Antiseptika sind auf Seite 4, Mitte, Formaldehyd oder Merthiolat genannt.

Die DE-A-2 603 122 beschreibt ein Verfahren zur Herstellung von Glykoproteinen durch Einwirkung von Hefefermenten auf natives Eiweiss in Gegenwart von Monosacchariden. Auf Seite 7, unten, heisst es, dass die Wirkung der Glykoproteine dann optimal ist, wenn ihr durchschnittliches Molekulargewicht zwischen 55 000 und 65 000 liegt. Die Struktur dieser Glykoproteine unterscheidet sich somit stark von derjenigen der in der vorliegenden Anmeldung genannten Muramylpeptide.

Die Erfindung betrifft in allererster Linie Antibiotikapräparate, welche dadurch gekennzeichnet sind, dass sie eines oder mehrere der vorgenannten Antibiotika und ein Muramylpeptid der Formel I enthalten.

Diese Präparate weisen die üblichen Mengen an Antibiotika, z. B. zwischen 50 und 1000 mg, bevorzugt zwischen etwa 200 und 500 mg auf, sowie 5 mg bis zur Hälfte der Antibiotikamenge an Muramylpeptid der Formel I. Diese Präparate können, insbesondere wenn sie für orale Gabe zu verwenden sind, ausserdem noch die üblichen Mengen an pharmakologischen Trägerstoffen, Streck- und/oder Verdünnungsmitteln enthalten.

Die hohe antibiotische Wirkung des neuen Verfahrens und der neuen Präparate kann durch «in vivo»-Versuche nachgewiesen werden, die an verschiedenen Tierarten, insbesondere an Säugetieren, wie Mäusen, durchgeführt werden. Dazu infiziert man sie mit einer letalen oder sub-letalen Dosis eines pathogenen Microorganismus und gibt dann das genannte neue Präparat bzw. die einzelnen Dosen an Muramylpeptid und Antibiotikum. Die Wirkung wird als $ED_{50}$ bestimmt, das ist diejenige Dosis bei denen 50% der Tiere überleben.

Es wurde nun überraschenderweise gefunden, dass die Infektion mit pathogenen Keimen, insbesondere der weniger beeinflussbaren gramnegativen Bakterien, wie z. B. Stämmen der Aerobakter, Brucella, Escherichia, Klebsiella, Malleomyces, Neisseria, Pasteurella, Proteus, Pseudomonas, Shigella und Vibrio, aber auch von grampositiven Bakterien, wie von Aktinomycetes, Clostridia, Corynebakterien, Diplokokken, Mycobakterien oder Staphylococcen, oder von Pilzen, wie Candida Albicans, Cryptokokkus neoformans, Plastomyces dermatitides oder Hystoplasma capsulatum, in erhöhtem Masse gehemmt und bekämpft wird.

Besonders hervorzuheben sind pharmazeutische oder veterinärmedizinische Präparate, sowie Futtermittel oder Futtermittelzusätze, die eine wirksame oder unterwirksame Dosis der genannten Antibiotika und zusätzlich ein Muramylpeptid der Formel I enthalten.

Die pharmazeutischen Präparate der vorliegenden Erfindung sind vorzugsweise Tabletten oder Gelatinekapseln, welche die Wirkstoffe zusammen mit Verdünnungsmitteln, z. B. Laktose, Dextrose, Saccharose, Mannit, Sorbit, Cellulose und/oder Glucose, und Schmiermitteln, z. B. Kieselerde, Talk, Stearinsäure oder Salze davon, wie Magnesium- oder Calciumstearat, und/oder Polyäthylenglykol, enthalten. Tabletten enthalten ebenfalls Bindemittel, z. B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, wie Natriumalginat und/oder Brausemischungen, oder Adsorptionsmittel, Farbstoffe, Geschmackstoffe und Süssmittel. Injizierbare Präparate sind vorzugsweise isotonische wässrige Lösungen oder Suspensionen. Suppositorien, Salben oder Cremen sind in erster Linie Fettemulsionen oder -suspensionen. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z. B. mittels konventioneller Misch-, Granulier- oder Dragierverfahren, hergestellt und enthalten von etwa 0,1% bis etwa 75%, insbesondere von etwa 1% bis etwa 50% der genannten Aktivstoffe.

Die oral applizierbaren Präparate der vorliegenden Erfindung können auch mit einem gegen

Magensaft beständigen Überzug versehen werden.

Die folgenden Beispiele dienen zur Illustration der Erfindung. Temperaturen werden in Celsiusgraden angegeben.

Beispiel 1:
Herstellung von 1000 Kapseln mit 260 mg der aktiven Ingredienzen pro Kapsel:

Zusammensetzung:

| | |
|---|---|
| Rifampicin | 250 g |
| N-Acetyl-muramyl-L-alanyl-Di-isoglutamin | 10 g |
| Talk | 36 g |
| Weizenstärke | 24 g |
| Magnesiumstearat | 16 g |
| Laktose | 4 g |
| | 340 g |

Zubereitung:
Die pulverförmigen Substanzen werden durch ein Sieb mit einer Maschenweite von 0,6 mm geschlagen und gründlich gemischt. Mit je 340 g dieser Mischung werden mit einer Kapselfüllmaschine Gelatine-Kapseln bereitet.

Beispiel 2:
Herstellung von 1000 Kapseln enthaltend 105 mg der aktiven Stoffe pro Kapsel:

Zusammensetzung:

| | |
|---|---|
| Rifampicin | 100 g |
| N-Acetyl-desmethylmuramyl-L-alanyl-D-isoglutamin | 5 g |
| Äthyl-Cellulose | 3 g |
| Stearinsäure | 3 g |
| | 111 g |

Zubereitung:
Die Äthyl-Cellulose und die Stearinsäure werden in 120 ml Methylenchlorid gelöst, mit dem Antibiotikum versetzt, und die Masse wird durch ein Sieb von 0,6 mm Maschenweite bei einer Temperatur von ca. 40° geschlagen, wobei das Methylenchlorid verdampft. 156 mg des erhaltenen Granulates werden in Gelatine-Kapseln zu 0,5 ml mit Hilfe einer Kapsel-Abfüllmaschine abgefüllt.

Beispiel 3:
Herstellung eines Futtermittels enthaltend 0,005% der aktiven Stoffe:

Vor-Mischung:

| | |
|---|---|
| Rifampicin oder Chlortetracyclin | 30 g |
| N-Benzoyl-6-0-stearoyl-desmethyl-muramyl-L-alanyl-D-isoglutamin | 10 g |
| Staubzucker | 50 g |
| Sojabohnen Futter (mit Lösungsmitteln extrahiert) | 275 g |
| | 365 g |

Zusatzstoffe:

| | |
|---|---|
| Maismehl | 500,0 kg |
| Sojabohnenmehl, 44% Protein | 300,0 kg |
| Alfalfamehl | 13,5 kg |
| Dicalciumphosphat | 18,0 kg |
| Calciumcarbonat (gemahlen) | 4,5 kg |
| Salz | 2,3 kg |
| Fischmehl, 60% Protein | 18,0 kg |
| Stab. Fett | 27,0 kg |
| trockener Molkerückstand | 18,0 kg |
| Mangansulfat | 0,2 kg |
| Zinkoxid | 1,3 kg |
| d,1-Methionin | 0,7 kg |
| Vitamin-Vormischung | 4,5 kg |
| | 908,0 kg |

Die Vitamin-Vormischung enthält in 4,5 kg:
16 000 000 I.E. Vit. A, 1 000 000 I.E. Vit. $D_3$, 5000 I.E. Vit. E Acetat, 6 g Vit. $K_3$, 6 mg Vit. $B_{12}$, 3 g Riboflavin, 30 g Niacin, 5 g Calcium Pantothenat und 100 g Ethoxyquin (1,2-Dihydro-6-äthoxy-2,2,4-trimethyl-chinolin) und Maismehl bis auf 4,5 kg.

Herstellungsweise:
Die Wirkstoffe und Zucker werden gründlich miteinander gemischt, durch ein Sieb von 0,6 mm Maschenweite geschlagen und dann mit dem Sojabohnenmehl vermischt. Die Vormischung wird dann in der der gewünschten Endkonzentration entsprechenden Menge zum Futtermittel zugegeben und in einem horizontalen Trommelmischer homogenisiert.

Beispiel 4:
Man infiziert weisse Laboratoriums-Mäuse auf i.p. Wege mit den in der nachfolgenden Tabelle angeführten pathogenen Mikroorganismen in Salzlösung, so dass 90–100% der nicht behandelten Kontrolltiere innerhalb von 48 Stunden getötet werden. Unmittelbar, sowie 3 Stunden nach der Infektion werden Gruppen zu je 10 Mäusen mit Cefroxadin, sowohl allein (ohne MDP oder nMDP) als auch in Kombination mit N-Acetyl-muramyl-L-alanyl-D-isoglutamin (MDP) bzw. mit N-Acetyl-desmethylmuramyl-L-alanyl-D-isoglutamin (nMDP) behandelt, wobei dieselben zweimal in Form wässriger Lösungen oder Suspensionen auf subkutanem Weg verabreicht wurden. Nach 4 bzw. 10 Tagen nach erfolgter Infektion wurden die überlebenden Tiere gezählt. Die meisten dieser Versuche wurden zweifach ausgeführt und die Mittelwerte ermittelt.

Die Resultate sind wie folgt:

| Infektionserreger cfu (colony forming units) i.p. (x $10^4$) | $ED_{50}$ [mg/kg] | | |
|---|---|---|---|
| | ohne MDP oder nMDP | mit 10 mg/kg MDP | mit 10 mg/kg nMDP |
| Klebsiella pneumoniae 329 (2) | 14,2 | 4,5 | $<$ 2,5 |
| Klebsiella pneumoniae 327 (60) | 75 | $<$50 | $<$50 |
| Pasteurella multocida (0,1) | 109 | 37 | 70 |
| Salmonella typhimurium 277 (a) (40) | 22,5 | 7,6 | 11 |
| Salmonella typhimurium 273 (a) (400) | 39 | 22,5 | 14 |
| Staphylococcus aureus 2999 (20 000) | 58 | 45 | 35 |
| Streptococcus pyogenes 38 (0,6) | 0,21 | 0,18 | 0,17 |

(a) Resultate nach 10 Tagen.

Beispiel 5:

In analoger Weise wie in den Beispielen 1 und 2 beschrieben erhält man Kombinationspräparate, die neben den Hilfs- und Trägerstoffen die folgenden aktiven Ingredienzien in den angegebenen Mengen pro Kapsel enthalten:

a) 500 mg Cephalexin und 5 mg 6-0-Stearoyl-N-acetyl-muramyl-L-alanyl-D-isoglutamin,

b) 750 mg Ampicillin und 40 mg N-Benzoyl-normuramyl-(α-methyl-alanyl)-D-isoglutamin,

c) 100 mg Doxycyclin und 15 mg N-Acetyl-muramyl-L-alanyl-D-glutaminsäure

d) 300 mg Methacyclin und 15 mg N-Benzoyl-desmethylmuramyl-L-alanyl-D-glutaminsäure-dimethylester,

e) 250 mg Erythromycin-Estolat und 30 mg N-Propionyl-desmethylmuramyl-L-α-aminobutyryl-glutaminsäure-diamid.

Beispiel 6:

Herstellung einer sterilen Trockensubstanz zur Injektion (Lyophilisation)

500 mg Cefsulodin und 10 mg N-Acetyl-muramyl-L-α-aminobutyryl-D-isoglutamin werden unter Rühren in 5 ml Wasser gelöst. Die Lösung wird sterilfiltriert und unter aseptischen Bedingungen in ein steriles Ampullenglas (Vial) abgefüllt und lyophilisiert. Die Trockensubstanz kann nach Auflösen in Wasser oder physiologischen Lösungen für die parenterale Applikation verwendet werden.

Beispiel 7:

Herstellung einer sterilen Trockensubstanz zur Injektion (Pulverabfüllung)

500 mg steriles Cefsulodin und 15 mg steriles N-Propionyl-desmethylmuramyl-L-alanyl-D-isoglutamin werden unter aseptischen Bedingungen homogen gemischt und in ein Ampullenglas abgefüllt. Die Trockensubstanz kann nach Auflösen in Wasser oder physiologischen Lösungen für die parenterale Applikation verwendet werden.

Beispiel 8:

In analoger Weise wie in den Beispielen 6 und 7 beschrieben erhält man sterile Trockensubstanzgemische zur Injektion, die folgende Mengen an wirksamen Bestandteilen enthalten:

a) 1000 mg Oxacillin (Natriumsalz) und 20 mg N-Acetyl-muramyl-L-α-aminobutyryl-glutaminsäure,

b) 500 mg Cefazolin und 20 mg N-Acetyl-desmethylmuramyl-L-prolyl-D-glutaminsäure-diäthylester,

c) 80 mg Gentamycin und 10 mg N-Benzoyldesmethylmuramyl-L-alanyl-D-glutaminsäure,

d) 200 mg Doxycyclin und 10 mg N-Propionyl-desmethylmuramyl-L-alanyl-D-isoglutamin,

e) 50 mg Amphotericin B und 41 mg Natrium-Desoxycholat sowie 5 mg 6-0-Stearoyl-N-acetyl-muramyl-L-alanyl-D-isoglutamin zur Herstellung der Stammlösung,

f) 500 mg Vancomycin und 6-0-Stearoyl-N-benzoyl-normuramyl-L-alanyl-D-isoglutamin.

**Patentansprüche**

1. Kombinationspräparat zur Anwendung in einem Verfahren zur Steigerung der antibiotischen Wirksamkeit von Antibiotika, das ein Antibiotikum aus der Gruppe der β-Lactamantibiotika, Aminoglycoside, Tetracycline, Macrolide, Lincomycine, Polyenantibiotika, Polypeptidantibiotika, Anthracycline, Chlor- oder Thiamphenicole, Cycloserine, Fusidinsäuren oder Rifamycine zusammen mit einem Muramylpeptid der Formel

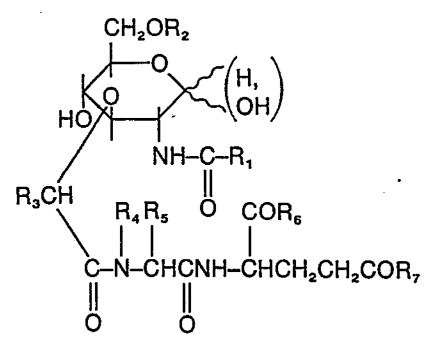

(I)

worin $R_1$ unsubstituiertes oder durch Hydroxy, Niederalkoxy, Niederalkanoyloxy, Halogen, Mercapto, Niederalkylthio, Amino oder Niederalkanoylamino substituiertes Niederalkyl oder Niederalkoxy, oder einen Phenyl- oder Phenylniederalkylrest, wobei der Phenylrest bzw. der Phenylteil gegebenenfalls durch Niederalkyl, Hydroxy, Niederalkoxy oder Halogen und der Niederalkylteil gegebenenfalls durch Hydroxy, Niederalkoxy, Niederalkanoyloxy oder Halogen substituiert ist,

$R_2$ Wasserstoff oder das Acylradikal einer aliphatischen Carbonsäure mit bis zu 30 C-Atomen,

$R_3$ Wasserstoff oder Niederalkyl,

$R_4$ Wasserstoff oder Niederalkyl,

$R_5$ Wasserstoff, unsubstituiertes oder durch Hydroxy, Niederalkoxy, Niederalkanoyloxy, Halogen, Mercapto, Niederalkylthio, Amino, Niederalkanoylamino oder gegebenenfalls durch Niederalkyl, Hydroxy, Niederalkoxy oder Halogen substituiertes Phenyl substituiertes Niederalkyl oder unsubstituiertes oder durch Niederalkyl, Hydroxy, Niederalkoxy oder Halogen substituiertes Phenyl, oder $R_4$ und $R_5$ zusammen Niederalkylen bedeuten, und jeder der Reste $R_6$ und $R_7$ für gegebenenfalls veräthertes Hydroxy oder gegebenenfalls durch Niederalkyl, welches unsubstituiert oder durch Hydroxy, Mercapto, Carboxy, Niederalkoxy, Niederalkylthio, Niederalkoxycarbonyl oder Carbamoyl substituiert ist, substituiertes Amino steht, wobei das Präfix «Nieder-» Reste bis und mit 7 C-Atomen bezeichnet, und/oder einem oder mehreren Salzen von Verbindungen der Formel I mit mindestens einer salzbildenden Gruppe enthält.

2. Kombinationspräparat gemäss Patentanspruch 1, das als Muramylpeptid ein solches der allgemeinen Formel II

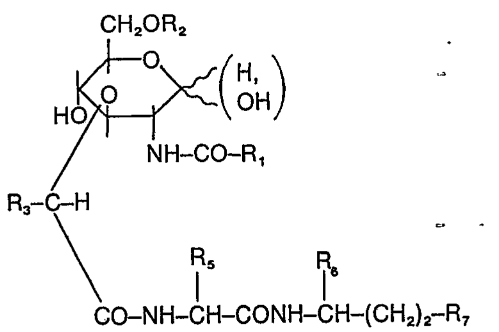

(II)

worin $R_1$ Niederalkyl oder Phenyl, $R_2$ Wasserstoff oder langkettiges Alkanoyl, $R_3$ Wasserstoff oder Methyl, $R_5$ Wasserstoff, Niederalkyl oder Hydroxymethyl, $R_6$ Carbamoyl und $R_7$ Carboxyl bedeuten, und/oder ein Salz davon enthält.

3. Kombinationspräparat gemäss Patentanspruch 2, das ein Muramylpeptid der in Anspruch 2 gegebenen Formel II, worin $R_1$ Methyl, $R_2$ Wasserstoff oder Stearoyl und $R_5$ Methyl bedeuten, und/oder ein Salz davon enthält.

4. Kombinationspräparat gemäss einem der Ansprüche 1–3, das als Antibiotikum 7β-[D-2-Amino-2-(1,4-cyclohexadienyl)-acetamido]-3-methoxy-3-cephem-4-carbonsäure enthält.

5. Pahrmazeutische Präparate mit verstärkter antibiotischer Wirkung, enthaltend eines oder mehrere Antibiotika aus der Gruppe der β-Lactamantibiotika, Aminoglycoside, Tetracycline, Macrolide, Lincomycine, Polyenantibiotika, Polypeptidantibiotika, Anthracycline, Chlor- oder Thiamphenicole, Cycloserine, Fusidinsäuren oder Rifamycine und ein Muramylpeptid der Formel

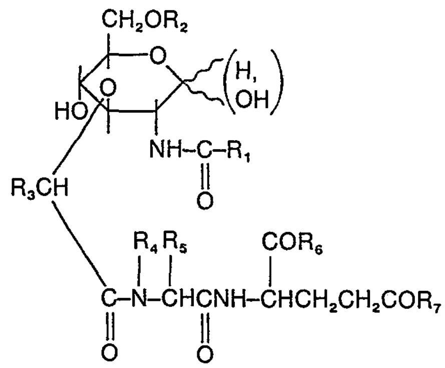

worin $R_1$ unsubstituiertes oder durch Hydroxy, Niederalkoxy, Niederalkanoyloxy, Halogen, Mercapto, Niederalkylthio, Amino oder Niederalkanoylamino substituiertes Niederalkyl oder Niederalkoxy, oder einen Phenyl- oder Phenylniederalkylrest, wobei der Phenylrest bzw. der Phenylteil gegebenenfalls durch Niederalkyl, Hydroxy, Niederalkoxy oder Halogen und der Niederalkylteil gegebenenfalls durch Hydroxy, Niederalkoxy, Niederalkanoyloxy oder Halogen substituiert ist,

$R_2$ Wasserstoff oder das Acylradikal einer aliphatischen Carbonsäure mit bis zu 30 C-Atomen,

$R_3$ Wasserstoff oder Niederalkyl,

$R_4$ Wasserstoff oder Niederalkyl,

$R_5$ Wasserstoff, unsubstituiertes oder durch Hydroxy, Niederalkoxy, Niederalkanoyloxy, Halogen, Mercapto, Niederalkylthio, Amino, Niederalkanoylamino oder gegebenenfalls durch Niederalkyl, Hydroxy, Niederalkoxy oder Halogen substituiertes Phenyl substituiertes Niederalkyl oder unsubstituiertes oder durch Niederalkyl, Hydroxy, Niederalkoxy oder Halogen substituiertes Phenyl, oder $R_4$ und $R_5$ zusammen Niederalkylen bedeuten, und jeder der Reste $R_6$ und $R_7$ für gegebenenfalls veräthertes Hydroxy oder gegebenenfalls durch Niederalkyl, welches unsubstituiert oder durch Hydroxy, Mercapto, Carboxy, Niederalkoxy, Niederalkylthio, Niederalkoxycarbonyl oder Carbamoyl substituiert ist, substituiertes Amino steht, wobei das Präfix «Nieder-» Reste bis und mit 7 C-Atomen bezeichnet, und/oder Salze von Verbindungen der Formel I mit mindestens einer salzbildenden Gruppe zusammen mit einem pharmazeutisch verwendbaren Trägermaterial.

6. Pharmazeutische Präparate gemäss Patentanspruch 5, dadurch gekennzeichnet, dass sie als Muramylpeptid ein solches der Formel

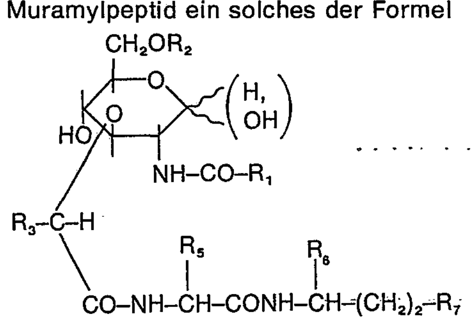

(II)

worin $R_1$ Niederalkyl oder Phenyl, $R_2$ Wasserstoff oder langkettiges Alkanoyl, $R_3$ Wasserstoff oder Methyl, $R_5$ Wasserstoff, Niederalkyl oder Hydroxymethyl, $R_6$ Carbamoyl und $R_7$ Carboxyl bedeuten, und/oder ein Salz davon enthalten.

7. Pharmazeutische Präparate gemäss Patentanspruch 6, dadurch gekennzeichnet, dass sie als Muramylpeptid ein solches der in Patentanspruch 6 gegebenen Formel worin $R_1$ Niederalkyl oder Phenyl, $R_2$ Wasserstoff, $R_3$ Wasserstoff oder Methyl, $R_5$ Wasserstoff, Methyl oder Hydroxymethyl, $R_6$ Carbamoyl und $R_7$ Carboxyl bedeuten, und/oder ein Salz davon enthalten.

8. Pharmazeutische Präparate gemäss Patentanspruch 6 oder 7, dadurch gekennzeichnet, dass sie ein Muramylpeptid der in Patentanspruch 6 angegebenen Formel II, worin $R_1$ Methyl, $R_2$ Wasserstoff oder Stearoyl, $R_3$ Wasserstoff oder Methyl, $R_5$ Methyl, $R_6$ Carbamoyl und $R_7$ Carboxy bedeuten, und/oder ein Salz davon enthalten.

9. Pharmazeutische Präparate gemäss einem der Patentansprüche 6–8, dadurch gekennzeichnet, dass sie als Antibiotikum Rifampicin, Chlortetracyclin, Cephalexin, Ampicillin, Doxycylin, Methacyclin, Erythromycin, Cefsulodin, Oxacillin, Cefazolin, Gentamycin, Amphotericin B oder Vancomycin enthalten.

10. Pharmazeutische Präparate gemäss einem der Patentansprüche 6–8, dadurch gekennzeichnet, dass sie als Antibiotikum die 7β-[D-2-Amino-2-(1,4-cyclohexadienyl)-acetamido]-3-methoxy-3-cephem-4-carbonsäure enthalten.

11. Futtermittel und Futtermittelzusätze, gekennzeichnet durch einen zusätzlichen Gehalt an einem oder mehreren der in Anspruch 1 genannten Antibiotika und einem der in Anspruch 1 genannten Muramylpeptide, und/oder einem oder mehreren Salzen dieser Muramylpeptide mit mindestens einer salzbildenden Gruppe.

12. Kombinationspräparat nach einem der Ansprüche 1 bis 3, das ein Antibiotikum aus der Gruppe der β-Lactamantibiotika, Lincomycine oder Rifamycine enthält.

13. Kombinationspräparat nach einem der Ansprüche 1 bis 3, das ein Antibiotikum aus der Gruppe der Polyenantibiotika enthält.

14. Kombinationspräparat nach Anspruch 12, das als Antibiotikum Rifampicin, Cefotiam oder Clindamycin enthält.

15. Kombinationspräparat nach Anspruch 13, das als Antibiotikum Amphotericin B enthält.

## Claims

1. A combination preparation for use in a method of intensifying the action of antibiotics, which preparation contains an antibiotic selected from the group consisting of the β-lactam antibiotics, aminoglycosides, tetracyclines, macrolides, lincomycins, polyene antibiotics, polypeptide antibiotics, anthracyclines, chloro- or thiamphenicols, cycloserins, fusidic acids or rifamycins, together with a muramylpeptide of the formula

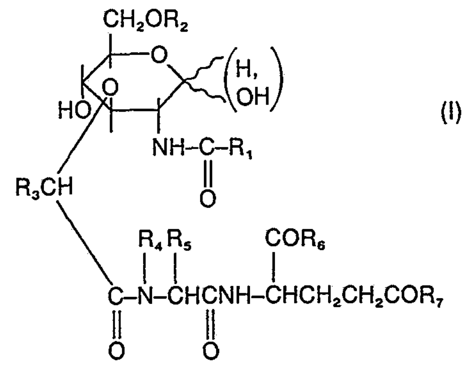

(I)

wherein

$R_1$ is lower alkyl or lower alkoxy, each of which is unsubstituted or substituted by hydroxy, lower alkoxy, lower alkanoyloxy, halogen, mercapto, lower alkylthio, amino or lower alkanoylamino, or is a phenyl or phenyl-lower alkyl radical, with phenyl being unsubstituted or substituted by lower alkyl, hydroxy, lower alkoxy or halogen, and lower alkyl being unsubstituted or substituted by hydroxy, lower alkoxy, lower alkanoyloxy or halogen,

$R_2$ is hydrogen or the acyl radical of an aliphatic carboxylic acid containing up to 30 carbon atoms,

$R_3$ is hydrogen or lower alkyl,

$R_4$ is hydrogen or lower alkyl,

$R_5$ is hydrogen, lower alkyl which is unsubstituted or substituted by hydroxy, lower alkoxy, lower alkanoyloxy, halogen, mercapto, lower alkylthio, amino, lower alkanoylamino or by phenyl which is unsubstituted or substituted by lower alkyl, hydroxy, lower alkoxy or halogen, or is phenyl which is unsubstituted or substituted by lower alkyl, hydroxy, lower alkoxy or halogen, or $R_4$ and $R_5$ together are lower alkylene, and

$R_6$ and $R_7$ are each free or etherified hydroxy, or are amino which is unsubstituted or substituted by lower alkyl which is unsubstituted or substituted by hydroxy, mercapto, carboxy, lower alkoxy, lower alkylthio, lower alkoxycarbonyl or carbamoyl, with the term «lower» denoting radicals containing up to and including 7 carbon atoms, and/or one or more salts of a compound of formula I containing at least one salt-forming group.

2. A combination preparation according to claim 1, which contains a muramylpeptide of the general formula II

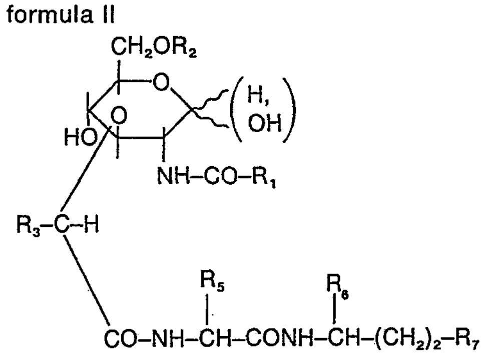

(II)

wherein $R_1$ is lower alkyl or phenyl, $R_2$ is hydrogen or longchain alkanoyl, $R_3$ is hydrogen or methyl, $R_5$ is hydrogen, lower alkyl or hydroxymethyl, $R_6$

is carbamoyl and $R_7$ is carboxyl, and/or salt thereof.

3. A combination preparation according to claim 2, which contains a muramylpeptide of the formula II as indicated in claim 2, wherein $R_1$ is methyl, $R_2$ is hydrogen or stearoyl and $R_5$ is methyl, and/or a salt thereof.

4. A combination preparation according to any one of claims 1 to 3, wherein the antibiotic is 7β-[D-2-amino-2-(1,4-cyclohexadienyl)acetamido]-3-methoxy-3-cephem-4-carboxylic acid.

5. A pharmaceutical preparation with intensified antibiotic action containing one or more antibiotics selected from the group consisting of the β-lactam antibiotics, aminoglycosides, tetracyclines, macrolides, lincomycins, polyene antibiotics, polypeptide antibiotics, anthracyclines, chloro- or thiamphenicols, cycloserins, fusidic acids or rifamycins, and a muramylpeptide of the formula

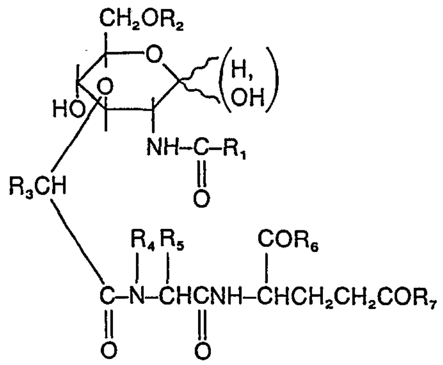

wherein

$R_1$ is lower alkyl or lower alkoxy, each of which is unsubstituted or substituted by hydroxy, lower alkoxy, lower alkanoyloxy, halogen, mercapto, lower alkylthio, amino or lower alkanoylamino, or is a phenyl or phenyl-lower alkyl radical, with phenyl being unsubstituted or substituted by lower alkyl, hydroxy, lower alkoxy or halogen, and lower alkyl being unsubstituted or substituted by hydroxy, lower alkoxy, lower alkanoyloxy or halogen,

$R_2$ is hydrogen or the acyl radical of an aliphatic carboxylic acid containing up to 30 carbon atoms,

$R_3$ is hydrogen or lower alkyl,

$R_4$ is hydrogen or lower alkyl,

$R_5$ is hydrogen, lower alkyl which is unsubstituted or substituted by hydroxy, lower alkoxy, lower alkanoyloxy, halogen, mercapto, lower alkylthio, amino, lower alkanoylamino or by phenyl which is unsubstituted or substituted by lower alkyl, hydroxy, lower alkoxy or halogen, or is phenyl which is unsubstituted or substituted by lower alkyl, hydroxy, lower alkoxy or halogen, or $R_4$ and $R_5$ together are lower alkylene, and

$R_6$ and $R_7$ are each free or etherified hydroxy, or are amino which is unsubstituted or substituted by lower alkyl which is unsubstituted or substituted by hydroxy, mercapto, carboxy, lower alkoxy, lower alkylthio, lower alkoxycarbonyl or carbamoyl, with the term «lower» denoting radicals containing up to and including 7 carbon atoms, and/or a salt of a compound of formula I containing at least one salt-forming group, together with a pharmaceutically acceptable carrier.

6. A pharmaceutical preparation according to claim 5 which contains a muramylpeptide of the formula

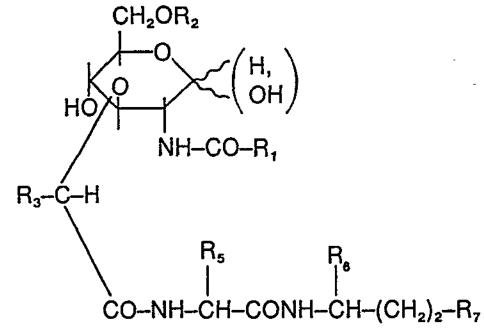

(II)

wherein $R_1$ is lower alkyl or phenyl, $R_2$ is hydrogen or longchain alkanoyl, $R_3$ is hydrogen or methyl, $R_5$ is hydrogen, lower alkyl or hydroxymethyl, $R_6$ is carbamoyl and $R_7$ is carboxyl, and/or a salt thereof.

7. A pharmaceutical preparation according to claim 6 which contains a muramylpeptide of the formula as indicated in claim 6, wherein $R_1$ is lower alkyl or phenyl, $R_2$ is hydrogen, $R_3$ is hydrogen or methyl, $R_5$ is hydrogen, methyl or hydroxymethyl, $R_6$ is carbamoyl and $R_7$ is carboxyl, and/or a salt thereof.

8. A pharmaceutical preparation according to either of claims 6 or 7 which contains a muramylpeptide of the formula II as indicated in claim 6, wherein $R_1$ is methyl, $R_2$ is hydrogen or stearoyl, $R_3$ is hydrogen or methyl, $R_5$ is methyl, $R_6$ is carbamoyl and $R_7$ is carboxyl, and/or a salt thereof.

9. A pharmaceutical preparation according to any one of claims 6 to 8, wherein the antibiotic is rifampicin, chlorotetracycline, cephalexin, ampicillin, doxcycline, methacycline, erythromycin, cefsulodin, oxacillin, cefazolin, gentamycin, amphotericin B or vancomycin.

10. A pharmaceutical preparation according to any one of claims 6 to 8, wherein the antibiotic is 7β-[D-2-amino-2-(1,4-cyclohexadienyl)acetamido]-3-methoxy-3-cephem-4-carboxylic acid.

11. An animal feedstuff or additive thereof additionally containing one or more of the antibiotics as indicated in claim 1 and a muramylpeptide as indicated in claim 1, and/or one or more salts of a muramylpeptide containing at least one salt-forming group.

12. A combination preparation according to any one of claims 1 to 3 which contains an antibiotic selected from the group consisting of the β-lactam antibiotics, lincomycins or rifamycins.

13. A combination preparation according to any one of claims 1 to 3 which contains an antibiotic selected from the group consisting of the polyene antibiotics.

14. A combination preparation according to claim 12, wherein the antibiotic is rifampicin, cefotiam or clindamycin.

15. A combination preparation according to claim 13, wherein the antibiotic is amphotericin B.

**Revendications**

1. Composition combinée pour application dans un procédé pour augmenter l'efficacité des antibiotiques, contenant un antibiotique du groupe des antibiotiques β-lactame, aminoglycosides, tétracyclines, macrolides, lincomycines, antibiotiques polyène, antibiotiques polypeptides, anthracyclines, chlor- ou thiamphénicoles, cyclosérines, acides fusidiques ou rifamycines avec un muramylpeptide de formule

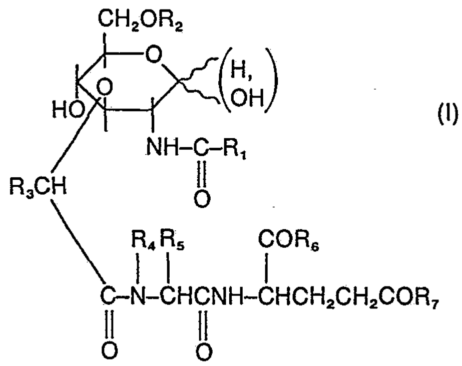

(I)

où $R_1$ représente un alcoyle inférieur ou un alcoxy inférieur non substitué ou substitué par un hydroxy, un alcoxy inférieur, un alcanoyloxy inférieur, un halogène, un mercapto, un alcoyle inférieur-thio, un amino ou un alcanoyle inférieur-amino, ou un radical phényle ou phényl-alcoyle inférieur, où le radical phényle ou selon les cas la fraction phényle est éventuellement substituée par un alcoyle inférieur, un hydroxy, un alcoxy inférieur ou un halogène et la fraction alcoyle inférieur est éventuellement substituée par un hydroxy, un alcoxy inférieur, un alcanoyloxy inférieur ou un halogène,

$R_2$ représente un hydrogène ou le radical acyle d'un acide carboxylique aliphatique ayant jusqu'à 30 atomes de carbone,

$R_3$ représente un hydrogène ou un alcoyle inférieur,

$R_4$ représente un hydrogène ou un alcoyle inférieur,

$R_5$ représente un hydrogène, un alcoyle inférieur non substitué ou substitué par un hydroxy, un alcoxy inférieur, un alcanoyloxy inférieur, un halogène, un mercapto, un alcoyle inférieur-thio, un amino, un alcanoyle inférieur-amino ou un phényle éventuellement substitué par un alcoyle inférieur, un hydroxy, un alcoxy inférieur ou un halogène, ou un phényle non substitué ou substitué par un alcoyle inférieur, un hydroxy, un alcoxy inférieur ou un halogène, ou $R_4$ et $R_5$ représentent ensemble un alcoylène inférieur, et chacun des radicaux $R_6$ et $R_7$ représente un hydroxy éventuellement éthérifié ou un amino éventuellement substitué par un alcoyle inférieur, qui est non substitué ou substitué par un hydroxy, un mercapto, un carboxy, un alcoxy inférieur, un alcoyle inférieur-thio, un alcoxy inférieur-carbonyle ou un carbamoyle, où le préfixe «inférieur» désigne des radicaux ayant jusqu'à 7 atomes de carbone compris, et/ou un ou plusieurs sels de composés de formule I ayant au moins un groupe salificateur.

2. Composition combinée selon la revendication 1, qui contient comme muramylpeptide un tel corps de formule générale II

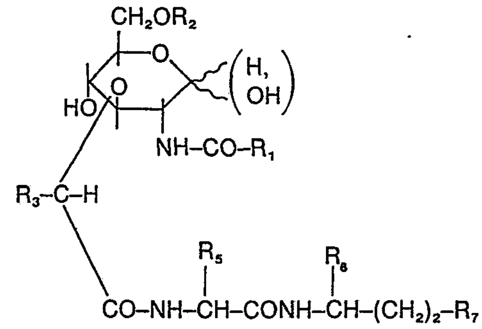

(II)

où $R_1$ représente un alcoyle inférieur ou un phényle, $R_2$ représente un hydrogène ou un alcanoyle à longue chaîne, $R_3$ représente un hydrogène ou un méthyle, $R_5$ représente un hydrogène, un alcoyle inférieur ou un hydroxyméthyle, $R_6$ représente un carbamoyle et $R_7$ représente un carboxyle, et/ou un de ses sels.

3. Composition combinée selon la revendication 2, qui contient un muramylpeptide de formule II donnée dans la revendication II, où $R_1$ représente un méthyle, $R_2$ un hydrogène ou un stéaroyle et $R_5$ représente un méthyle, et/ou un de ses sels.

4. Composition combinée selon l'une des revendications 1–3, qui contient comme antibiotique l'acide 7β-[D-2-amino-2-(1,4-cyclohexadiényl)-acétamido]-3-méthoxy-3-cephem-4-carboxylique.

5. Préparations pharmaceutiques à action antibiotique renforcée, contenant un ou plusieurs antibiotiques du groupe des antibiotiques β-lactame, aminoglycosides, tétracyclines, macrolides, lincomycines, antibiotiques polyène, antibiotiques polypeptides, anthracyclines, chlor- ou thiamphénicoles, cyclosérines, acides fusidiques ou rifamycines et un muramylpeptide de formule

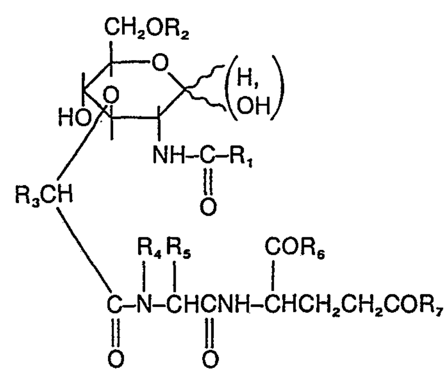

où $R_1$ représente un alcoyle inférieur ou un alcoxy inférieur non substitué ou substitué par un hydroxy, un alcoxy inférieur, un alcanoyloxy inférieur, un halogène, un mercapto, un alcoyle inférieur-thio, un amino ou un alcanoyle inférieur-amino, ou un radical phényle ou phényl-alcoyle inférieur, où le radical phényle ou selon les cas la fraction phényle est éventuellement substituée(e) par un alcoyle inférieur, un hydroxy, un alcoxy

inférieur ou un halogène, et la fraction alcoyle inférieur est éventuellement substituée par un hydroxy, un alcoxy inférieur, un alcanoyloxy inférieur ou un halogène,

$R_2$ représente un hydrogène ou le radical acyle d'un acide carboxylique aliphatique ayant jusqu'à 30 atomes de carbone,

$R_3$ représente un hydrogène ou un alcoyle inférieur,

$R_4$ représente un hydrogène ou un alcoyle inférieur,

$R_5$ représente un hydrogène, un alcoyle inférieur non substitué ou substitué par un hydroxy, un alcoxy inférieur, un alcanoyloxy inférieur, un halogène, un mercapto, un alcoyle inférieur-thio, un amino, un alcanoyle inférieur-amino ou un phényle éventuellement substitué par un alcoyle inférieur, un hydroxy, un alcoxy inférieur ou un halogène ou un phényle non substitué ou substitué par un alcoyle inférieur, un hydroxy, un alcoxy inférieur ou un halogène, ou $R_4$ et $R_5$ représentent ensemble un alcoylène inférieur, et chacun des radicaux $R_6$ et $R_7$ représente un hydroxy éventuellement éthérifié ou un amino éventuellement substitué par un alcoyle inférieur, qui est non substitué ou substitué par un hydroxy, un mercapto, un carboxy, un alcoxy inférieur, un alcoyle inférieur-thio, un alcoxy inférieur-carbonyle ou un carbamoyle, où le préfixe «inférieur» désigne des radicaux ayant jusqu'à 7 atomes de carbone compris, et/ou les sels des composés de formule I avec au moins un groupe salificateur, joints à un support pharmaceutiquement acceptable.

6. Préparations pharmaceutiques selon la revendication 5, caractérisées en ce qu'elles contiennent comme muramylpeptide un tel corps de formule

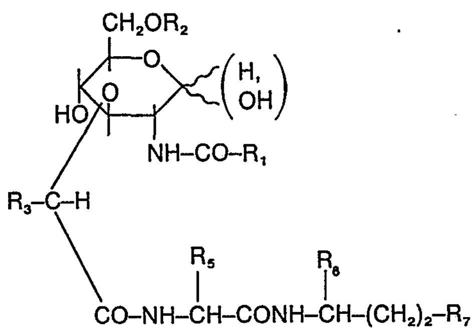

(II)

où $R_1$ représente un alcoyle inférieur ou un phényle, $R_2$ représente un hydrogène ou un alcanoyle à longue chaîne, $R_3$ représente un hydrogène ou un méthyle, $R_5$ représente un hydrogène, un alcoyle inférieur ou un hydroxyméthyle, $R_6$ représente un carbamoyle et $R_7$ un carboxyle, et/ou un de ses sels.

7. Préparations pharmaceutiques selon la revendication 6, caractérisées en ce qu'elles contiennent comme muramylpeptide un tel corps de formule donnée dans la revendication 6, où $R_1$ représente un alcoyle inférieur ou un phényle, $R_2$ un hydrogène, $R_3$ un hydrogène ou un méthyle, $R_5$ un hydrogène, un méthyle ou un hydroxyméthyle, $R_6$ un carbamoyle et $R_7$ un carboxyle, et/ou un de ses sels.

8. Préparations pharmaceutiques selon les revendications 7 et 8, caractérisées en ce qu'elles contiennent un muramylpeptide de formule II donnée dans la revendication 6, où $R_1$ représente un méthyle, $R_2$ un hydrogène ou un stéaroyle, $R_3$ un hydrogène ou un méthyle, $R_5$ un méthyle, $R_6$ un carbamoyle et $R_7$ un carboxy, et/ou un de ses sels.

9. Préparations pharmaceutiques selon l'une des revendications 6–8, caractérisées en ce qu'elles contiennent comme antibiotique la rifampicine, la chlorotétracycline, la céphalexine, l'ampicilline, la doxycycline, la méthacycline, l'érythromycine, la cefsulodine, l'oxacilline, la céfazoline, la gentamycine, l'amphotéricine B ou la vancomycine.

10. Préparations pharmaceutiques selon l'une des revendications 6–8, caractérisées en ce qu'elles contiennent comme antibiotique l'acide 7β-[D-2-amino-2-(1,4-cyclohexadiényl)-acétamido]-3-méthoxy-3-cephem-4-carboxylique.

11. Aliments du bétail et additifs pour aliments du bétail, caractérisés en ce qu'ils contiennent en outre un ou plusieurs des antibiotiques mentionnés dans la revendication 1 et un des muramylpeptides mentionnés dans la revendication 1, et/ou un ou plusieurs sels de ces muramylpeptides avec au moins un groupe salificateur.

12. Composition combinée selon l'une des revendications 1 à 3, contenant un antibiotique du groupe des antibiotiques β-lactame, de la lincomycine ou de la rifamycine.

13. Composition combinée selon l'une des revendications 1 à 3, contenant un antibiotique du groupe des antibiotiques polyène.

14. Composition combinée selon la revendication 12, contenant comme antibiotique la rifampicine, le cefotiam ou la clindamycine.

15. Composition combinée selon la revendication 13, contenant comme antibiotique l'amphotéricine B.